(19) Europäisches Patentamt
European Patent Office
Office européen des brevets

(11) EP 3 447 047 A1

(12) EUROPEAN PATENT APPLICATION
published in accordance with Art. 153(4) EPC

(43) Date of publication:
27.02.2019 Bulletin 2019/09

(21) Application number: 17786062.4

(22) Date of filing: 21.04.2017

(51) Int Cl.:
*C07D 213/61* (2006.01) *A61K 31/44* (2006.01)
*A61P 13/10* (2006.01)

(86) International application number:
PCT/JP2017/016109

(87) International publication number:
WO 2017/183725 (26.10.2017 Gazette 2017/43)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 22.04.2016 JP 2016086239

(71) Applicant: ONO Pharmaceutical Co., Ltd.
Osaka-shi, Osaka 541-8526 (JP)

(72) Inventors:
- YASHIRO, Kentaro
Mishima-gun
Osaka 618-8585 (JP)
- KIJIMA, Hideomi
Mishima-gun
Osaka 618-8585 (JP)
- WAKAMATSU, Daisuke
Mishima-gun
Osaka 618-8585 (JP)
- SAITO, Tetsuji
Mishima-gun
Osaka 618-8585 (JP)

(74) Representative: Jones, Nicholas Andrew
Withers & Rogers LLP
4 More London Riverside
London, SE1 2AU (GB)

# (54) CRYSTAL POLYMORPHISM OF KCNQ 2-5 CHANNEL ACTIVATOR

(57) Crystal polymorphism may exist in a crystalline compound. In the case where crystal polymorphism exists, depending on the crystal form, solubility, dissolution rate, stability against heat, light, humidity, etc. or the like is different. Accordingly, in the production of a pharmaceutical product, it is a very important task to select a crystal form of a drug substance most suitable for a disease indication and a dosage form. The present invention relates to novel crystal forms (A crystal, W crystal, and a hydrate crystal (H crystal)) of a compound I having a strong opening action with respect to KCNQ2-5 channels.

EP 3 447 047 A1

## Description

TECHNICAL FIELD

The present invention relates to a novel crystal form of 1-[(5-chloro-2-pyridinyl)methyl]-3-{2,6-dichloro-4-[(2S)-1,1,1-trifluoro-2-hydroxy-2-propanyl]phenyl}urea (hereinafter sometimes abbreviated as compound I).

BACKGROUND ART

It has been confirmed that a KCNQ channel has five subtypes including KCNQ1, KCNQ2, KCNQ3, KCNQ4, and KCNQ5. Among them, KCNQ2-5 other than KCNQ1 are expressed in the nociceptive sensory system such as spinal dorsal root ganglion and spinal cord. The activation of the KCNQ2-5 channels causes hyperpolarization of nerve cells in a nociceptive signal pathway.

It has been reported that a KCNQ2-5 channel activator is useful for treating many disorders characterized by abnormal neuronal excitability including epilepsy, pain, migraine, and anxiety disorder (see Non-Patent Document 1). In fact, retigabine which is a KCNQ2-5 channel activator has already been marketed as an antiepileptic drug.

Further, it has also been recently reported that retigabine is useful for treating bladder disorder (overactive bladder or the like) (see Non-Patent Documents 2 and 3).

Overactive bladder is considered to be caused by a state of potential overactivity of the detrusor muscle, and therefore, a muscarinic receptor antagonist mainly having a suppressive action on bladder contraction has been widely used for its treatment. However, the muscarinic receptor is present not only in the bladder but also in the salivary gland, the intestinal tract, the ciliary muscle, and the like, and the muscarinic receptor has also a functional role. Therefore, adverse effects such as dry mouth, constipation, and blurred vision sometimes occur, and also there is a concern that the suppressive action on bladder contraction of the muscarinic receptor antagonist may cause adverse effects such as difficulty in urination, an increase in the amount of residual urine, and urinary retention, and it cannot be said that a satisfactory therapeutic effect is always achieved. Further, as a drug to overcome the problems of the muscarinic receptor antagonist, a selective β3 adrenergic receptor agonist was put on the market in 2011 in Japan. It is suggested that the selective β3 adrenergic receptor agonist hardly affects the urination function while enhancing the urine collection function by a bladder relaxing action, and it exhibits the bladder relaxing action independent of contraction stimulation, and therefore is expected to work on a wide range of patients. On the other hand, the risk of QT extension increases with an increase in the dose and it shows a heart rate increasing action by acting on the cardiac β receptor, and therefore, these are limiting factors for the dose.

From the above, in this field, a drug which has a bladder relaxing action independent of contraction stimulation and is free from fear of adverse effects has been demanded, and a KCNQ2-5 channel activator is expected as a drug which responds to these unmet medical needs.

Until now, as a KCNQ activator having a monocyclic amide skeleton, for example, a compound represented by the general formula (a) is known (see Patent Document 1).

[Chem. 1]

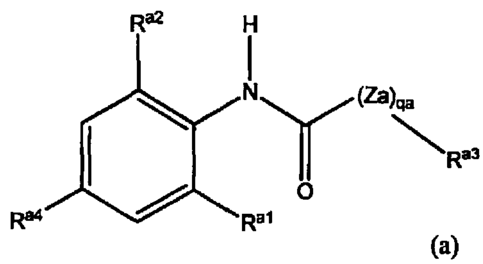

(a)

(wherein Za is O or S; qa is 0 or 1; $R^{a1}$ and $R^{a2}$ are each independently selected from the group consisting of halogen, cyano, amino, $C_{1-6}$-alkyl (alkenyl/alkynyl), and the like; $R^{a3}$ is selected from the group consisting of $C_{1-8}$-alkyl (alkenyl/alkynyl), $C_{3-8}$-cycloalkyl (cycloalkenyl), $C_{3-8}$-cycloalkyl(cycloalkenyl)-$C_{1-6}$-alkyl(alkenyl/alkynyl), aryl-$C_{1-6}$-alkyl (alkenyl/alkynyl), aryl-$C_{3-8}$-cycloalkyl(cycloalkenyl), and the like; and $R^{a4}$ is selected from the group consisting of halogen, cyano, $C_{1-6}$-alkyl(alkenyl/alkynyl), $C_{3-8}$-cycloalkyl (cycloalkenyl), $C_{3-8}$-cycloalkyl(cycloalkenyl)-$C_{1-6}$-alkyl(alkenyl/alky-

nyl), and the like (the definitions of the groups were partially extracted). The compound I is not included in the general formula (a) of Patent Document 1. Further, Patent Document 1 does not describe or suggest a technique for achieving the compound I from the compound described in Patent Document 1.

Further, in WO 2016/063990 (hereinafter sometimes abbreviated as Patent Application 2), the compound I is described as a KCNQ activator. However, Patent Application 2 does not describe that crystal polymorphism exists in the compound I.

CITED REFERENCES

PATENT DOCUMENTS

Patent Document 1: International Publication number WO 2006/029623
Patent Document 2: International Publication number WO 2016/063990

NON-PATENT DOCUMENTS

Non-Patent Document 1: Current Topics in Medicinal Chemistry, vol. 6, pp. 999-1023, 2006
Non-Patent Document 2: The Journal of Urology, vol. 172, pp. 2054-2058, 2004
Non-Patent Document 3: European Journal of Pharmacology, vol. 638, pp. 121-127, 2010

DISCLOSURE OF THE INVENTION

PROBLEMS TO BE SOLVED BY THE INVENTION

An object of the present invention is to provide a compound having a strong opening action with respect to KCNQ2-5 channels.

Further, crystal polymorphism may exist in a crystalline compound. In the case where crystal polymorphism exists, depending on the crystal form, solubility, dissolution rate, stability against heat, light, humidity, etc. or the like is different. Accordingly, in the production of a pharmaceutical product, it is a very important task to select a crystal form of a drug substance most suitable for a disease indication and a dosage form.

MEANS FOR SOLVING THE PROBLEMS

The present inventors found that other than the crystal form (M crystal) of the compound I produced in Example 25(5) of the above-mentioned Patent Application 2, other crystal forms exist in the compound I. Therefore, when they made intensive studies of the crystal polymorphism of the compound I, A crystal and W crystal were identified as novel crystal forms of the compound I, and further, a crystal of a hydrate (H crystal) of the compound I was identified (these novel crystal forms are sometimes abbreviated as "crystal form of the present invention").

That is, the present invention relates to:

(1) a crystal of 1-[(5-chloro-2-pyridinyl)methyl]-3-{2,6-dichloro-4-[(2S)-1,1,1-trifluoro-2-hydroxy-2-propanyl]phenyl} urea, having at least peaks of about 15.3, 16.9, 17.4, 19.3, and 19.6 °2θ in a powder X-ray diffraction spectrum;
(2) the crystal of 1-[(5-chloro-2-pyridinyl)methyl]-3-{2,6-dichloro-4-[(2S)-1,1,1-trifluoro-2-hydroxy-2-propanyl]phenyl}urea according to the above (1), having no peaks of about 7.0 and 9.2 °2θ in a powder X-ray diffraction spectrum;
(3) the crystal of 1-[(5-chloro-2-pyridinyl)methyl]-3-{2,6-dichloro-4-[(2S)-1,1,1-trifluoro-2-hydroxy-2-propanyl]phenyl}urea according to the above (1) or (2), having peaks of about 11.3, 11.6, 11.9, 12.8, 13.9, 15.3, 16.4, 16.9, 17.4, 18.1, 19. 3, 19.6, 20.5, 21.1, 22.2, 22.8, 23.5, 23.8, 24.3, and 24.7 °2θ in a powder X-ray diffraction spectrum;
(4) the crystal of 1-[(5-chloro-2-pyridinyl)methyl]-3-{2,6-dichloro-4-[(2S)-1,1,1-trifluoro-2-hydroxy-2-propanyl]phenyl}urea according to any one of the above (1) to (3), characterized by a powder X-ray diffraction spectrum chart shown in FIG. 3;
(5) a crystal of 1-[(5-chloro-2-pyridinyl)methyl]-3-{2,6-dichloro-4-[(2S)-1,1,1-trifluoro-2-hydroxy-2-propanyl]phenyl} urea, having an endothermic peak with an onset temperature of about 166°C or a peak temperature of about 167°C in differential scanning calorimetry;
(6) the crystal of 1-[(5-chloro-2-pyridinyl)methyl]-3-{2,6-dichloro-4-[(2S)-1,1,1-trifluoro-2-hydroxy-2-propanyl]phenyl}urea according to the above (5), characterized by a differential scanning calorimetry chart shown in FIG. 4;

(7) a crystal of a hydrate of 1-[(5-chloro-2-pyridinyl)methyl]-3-{2,6-dichloro-4-[(2S)-1,1,1-trifluoro-2-hydroxy-2-propanyl]phenyl}urea;

(8) the crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to the above (7), having at least peaks of about 13.0, 14.5, 18.3, 19.0, and 21.7 °2θ in a powder X-ray diffraction spectrum;

(9) the crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to the above (8), having no peaks of about 7.0 and 9.2 °2θ in a powder X-ray diffraction spectrum;

(10) the crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to the above (8) or (9), having peaks of about 6.5, 8.6, 11.4, 11.7, 13.0, 14.5, 15.1, 16.3, 18.3, 19.0, 19.4, 20.1, 20.5, 21.7, 21.9, 22.3, 23.0, 23.5, 23.8, 24.2, and 24.5 °2θ in a powder X-ray diffraction spectrum;

(11) the crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to any one of the above (8) to (10), characterized by a powder X-ray diffraction spectrum chart shown in FIG. 7;

(12) the crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to the above (7), having an endothermic peak with an onset temperature of about 32°C or a peak temperature of about 48°C in differential scanning calorimetry;

(13) the crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to the above (12), characterized by a differential scanning calorimetry chart shown in FIG. 8;

(14) a crystal of 1-[(5-chloro-2-pyridinyl)methyl]-3-{2,6-dichloro-4-[(2S)-1,1,1-trifluoro-2-hydroxy-2-propanyl]phenyl}urea, having peaks of about 10.6, 15.2, 19.6, 20.7, 22.4, and 24.1 °2θ in a powder X-ray diffraction spectrum;

(15) the crystal of 1-[(5-chloro-2-pyridinyl)methyl]-3-{2,6-dichloro-4-[(2S)-1,1,1-trifluoro-2-hydroxy-2-propanyl]phenyl}urea according to the above (14), having no peaks of about 7.0 and 9.2 °2θ in a powder X-ray diffraction spectrum;

(16) the crystal of 1-[(5-chloro-2-pyridinyl)methyl]-3-{2,6-dichloro-4-[(2S)-1,1,1-trifluoro-2-hydroxy-2-propanyl]phenyl}urea according to the above (14) or (15), characterized by a powder X-ray diffraction spectrum chart shown in FIG. 5;

(17) the crystal of 1-[(5-chloro-2-pyridinyl)methyl]-3-{2,6-dichloro-4-[(2S)-1,1,1-trifluoro-2-hydroxy-2-propanyl]phenyl}urea according to any one of the above (14) to (16), having an endothermic peak with an onset temperature of about 89°C or a peak temperature of about 94°C in differential scanning calorimetry;

(18) the crystal of 1-[(5-chloro-2-pyridinyl)methyl]-3-{2,6-dichloro-4-[(2S)-1,1,1-trifluoro-2-hydroxy-2-propanyl]phenyl}urea according to the above (17), characterized by a differential scanning calorimetry chart shown in FIG. 6;

(19) a pharmaceutical composition comprising the crystal according to any one of the above (1) to (18) and a pharmaceutically acceptable carrier;

(20) the pharmaceutical composition according to the above (19), which is a prophylactic and/or therapeutic agent for a KCNQ2-5 channel-related disease;

(21) the pharmaceutical composition according to the above (20), wherein the KCNQ2-5 channel-related disease is dysuria;

(22) the pharmaceutical composition according to the above (21) wherein the dysuria is overactive bladder;

(23) a prophylactic and/or therapeutic agent for a KCNQ2-5 channel-related disease, comprising the crystal according to any one of the above (1) to (18);

(24) a method for preventing and/or treating a KCNQ2-5 channel-related disease, characterized by administering an effective amount of the crystal according to any one of the above (1) to (18) to a mammal;

(25) the crystal according to any one of the above (1) to (18) for preventing and/or treating a KCNQ2-5 channel-related disease;

(26) use of the crystal according to any one of the above (1) to (18) for manufacturing a prophylactic and/or therapeutic agent for a KCNQ2-5 channel-related disease; and the like.

## EFFECT OF THE INVENTION

The compound I is useful as a prophylactic and/or therapeutic agent for a KCNQ2-5 channel-related disease. Further, the crystal form of the present invention is useful as a drug substance of a pharmaceutical product.

## BRIEF DESCRIPTION OF THE DRAWINGS

Fig. 1 shows a powder X-ray diffraction spectrum chart of M crystal of the compound I.
Fig. 2 shows a differential scanning calorimetry (DSC) chart of the M crystal of the compound I.
Fig. 3 shows a powder X-ray diffraction spectrum chart of A crystal of the compound I.
Fig. 4 shows a DSC chart of the A crystal of the compound I.
Fig. 5 shows a powder X-ray diffraction spectrum chart of W crystal of the compound I.
Fig. 6 shows a DSC chart of the W crystal of the compound I.
Fig. 7 shows a powder X-ray diffraction spectrum chart of a crystal of a hydrate (H crystal) of the compound I.
Fig. 8 shows a DSC chart of the crystal of a hydrate (H crystal) of the compound I.
Fig. 9 shows a water vapor adsorption/desorption isotherm of the crystal of a hydrate (H crystal) of the compound I. The horizontal axis represents a relative humidity (RH (%)), and the vertical axis represents a percent change in mass(Change in Mass (%)-Ref) at each humidity based on the weight when dried (0% RH).
Fig. 10 shows powder X-ray diffraction-differential scanning calorimetry simultaneous measurement of the crystal of a hydrate (H crystal) of the compound I.

EMBODIMENTS FOR CARRYING OUT THE INVENTION

In the present invention, the compound I is a compound represented by the following structure.

[Chem. 2]

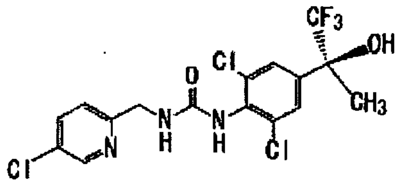

In the present invention, as novel crystal forms of the compound I, two types of anhydrous crystals: A crystal and W crystal, and a crystal of a hydrate: H crystal were identified. Differences among the crystal forms are distinguished by particularly powder X-ray diffraction spectra or/and differential scanning calorimetry (DSC).

The A crystal of the compound I is characterized by physicochemical data of at least one of the following (a) and (b). Preferably, it is characterized by physicochemical data of both (a) and (b): (a) a powder X-ray diffraction spectrum chart shown in the following FIG. 3, diffraction angles (2θ) shown in the following Table 2, or having at least peaks of about 15.3, 16.9, 17.4, 19.3, and 19.6 °2θ (further preferably, having no peaks of about 7.0 and/or 9.2 °2θ) in a powder X-ray diffraction spectrum; and (b) a DSC chart shown in the following FIG. 4 or having an endothermic peak with an onset temperature of about 166°C and/or a peak temperature of about 167°C in DSC.

The W crystal of the compound I is characterized by physicochemical data of at least one of the following (c) and (d). Preferably, it is characterized by physicochemical data of both (c) and (d): (c) a powder X-ray diffraction spectrum chart shown in the following FIG. 5 or diffraction angles (2θ) shown in the following Table 3 (further preferably, having no peaks of about 7.0 and/or 9.2 °2θ); and (d) a DSC chart shown in the following FIG. 6 or having an endothermic peak with an onset temperature of about 89°C and/or a peak temperature of about 94°C in DSC.

The H crystal which is a crystal of a hydrate of the compound I is characterized by physicochemical data of at least one of the following (e) and (f). Preferably, it is characterized by physicochemical data of both (e) and (f): (e) a powder X-ray diffraction spectrum chart shown in the following FIG. 7, diffraction angles (2θ) shown in the following Table 4, or having at least peaks of about 13.0, 14.5, 18.3, 19.0, and 21.7 °2θ (further preferably, having no peaks of about 7.0 and/or 9.2 °2θ) in a powder X-ray diffraction spectrum; and (f) a DSC chart shown in the following FIG. 8 or having an endothermic peak with an onset temperature of about 32°C and/or a peak temperature of about 48°C in DSC.

On the other hand, the M crystal of the compound I described in the above-mentioned Patent Application 2 is characterized by physicochemical data of (g) a powder X-ray diffraction spectrum chart shown in FIG. 1 or diffraction angles (2θ) shown in the following Table 1 and/or (h) a DSC chart (onset temperature: about 90°C, peak temperature: about 100°C) shown in FIG. 2.

In the present invention, the respective crystal forms of the compound I are specified by the physicochemical data described in this specification, however, the respective spectrum data can slightly vary due to their nature, and therefore should not be strictly construed.

For example, in the powder X-ray diffraction spectrum data, the diffraction angle (2θ) or the overall pattern is important for determination of the identity of the crystals due to their nature, and the relative intensity can slightly vary

depending on the crystal growth direction, the grain size, and the measurement conditions.

Further, also in the DSC data, the overall pattern is important for determination of the identity of the crystals and can slightly vary depending on the measurement conditions.

Therefore, those showing a powder X-ray diffraction spectrum pattern or a DSC pattern which is similar as a whole to that of the crystal form of the present invention are included in the crystal form of the present invention.

In this specification, the description of the diffraction angle (2θ (°)) in the powder X-ray diffraction spectrum pattern and the onset temperature (°C) and the peak temperature (°C) of the endothermic peak in the DSC analysis means that they include error ranges generally allowed in the data measurement methods, and means that they are approximately the diffraction angle, and the onset temperature and the peak temperature of the endothermic peak. For example, the word "about" attached to the diffraction angle (2θ (°)) in the powder X-ray diffraction is ±0.2° in a certain embodiment, and ±0.1° in another embodiment. The word "about" attached to the onset temperature (°C) or the peak temperature (°C) of the endothermic peak in the DSC analysis is ±2°C in a certain embodiment, and ±1°C in another embodiment. Further, the word "about" before the first number is also applied to the numbers thereafter. For example, the phrase "peaks of about 15.3, 16.9, 17.4, 19.3, and 19.6 °2θ means "peaks of about 15.3, about 16.9, about 17.4, about 19.3, and about 19.6 °2θ".

In one embodiment of the present invention, each crystal form of the compound I is substantially pure. The reference to "substantially pure" means that a specific crystal form accounts for at least 50% of the existing compound. Further, in another embodiment, each crystal form accounts for at least 75%, at least 85%, at least 90%, at least 95%, or about 94% to 98% of the existing compound I.

In the present invention, examples of the crystal of a hydrate of the compound I include 0.5 hydrate to 5 hydrate. In one embodiment of the present invention, the hydrate is 0.5 hydrate, 1 hydrate, 1.5 hydrate, 2 hydrate, or 2.5 hydrate. In one embodiment of the present invention, the hydrate is 0.5 hydrate to 1.0 hydrate, and in a specific embodiment, it is 0.5, 0.6, 0.7, 0.8, 0.9, or 1 hydrate.

In the present invention, the hydrate is not particularly limited as long as it is a crystal which stably holds an equivalent amount of water in an environment (temperature, relative humidity, etc.) where a pharmaceutical product is generally stored and used. For example, here, 1 hydrate is a crystal which stably holds 1 equivalent of water in an environment (temperature, relative humidity, etc.) where a pharmaceutical product is generally stored and used.

In the present invention, the crystal form of the present invention can be produced according to, for example, a method shown below, a method equivalent thereto, or Examples. Incidentally, when recrystallization is performed, a seed crystal may be used or may not be used.

The A crystal of the compound I can be produced, for example, from the compound I produced in the same manner as in Example 25(5) of Patent Application 2 by, for example, the following method.

The compound I is dissolved in a solvent (for example, ethanol) or a mixed solvent (for example, a mixed solvent of ethanol and water), followed by cooling, whereby the A crystal of the compound I can be obtained.

The H crystal which is the crystal of a hydrate of the compound I can be produced, for example, from the compound I produced in the same manner as in Example 25(5) of Patent Application 2 or the compound I produced in the same manner as in the below-mentioned Example 10 by, for example, the following method.

The compound I is added to a solvent (for example, water) or a mixed solvent (for example, a mixed solvent of acetone and water), followed by stirring at 25 to 40°C for 1 week or more, whereby the H crystal which is the crystal of a hydrate of the compound I can be obtained.

Alternatively, the compound I is dissolved in a mixed solvent of acetone and water, followed by cooling, whereby the H crystal which is the crystal of a hydrate of the compound I can be obtained.

The W crystal of the compound I can be produced, for example, from the compound I produced in the same manner as in Example 25(5) of Patent Application 2 or the below-mentioned Example 10 by, for example, the following method.

The compound I is dissolved in a solvent (for example, methanol) or a mixed solvent (for example, a mixed solvent of methanol and water) at 40 to 60°C, followed by cooling, whereby the W crystal of the compound I can be obtained.

Alternatively, the compound I is added to a solvent (for example, methanol) or a mixed solvent (for example, a mixed solvent of methanol and water), followed by stirring at room temperature for 8 hours or more, whereby the W crystal of the compound I can be obtained.

[Toxicity]

The toxicity of the compound I is sufficiently low and can be safely used as a pharmaceutical product.

[Application to Pharmaceutical Product]

The compound I is suitable for preventing and/or treating a KCNQ2-5 channel-related disease.

The compound I can be used for preventing and/or treating a KCNQ2-5 channel-related disease. Examples of such a disease include epilepsy, pain disorders (for example, neuropathic pain and migraine), diabetic peripheral neuropathy, anxiety disorder, mood adjustment disorder, schizophrenic disorder, drug dependence, attention adjustment disorder, sleep disorder, cerebral stroke, tinnitus, memory impairments (for example, Alzheimer's disease and dementia), amyotrophic lateral sclerosis, movement disorders (for example, Parkinson's disease, and dystonia-related movement disorder), dysuria (for example, overactive bladder, frequent urination, nocturia, urinary urgency, urge urinary incontinence, stress urinary incontinence, interstitial cystitis, chronic prostatitis, and prostatic hyperplasia), hearing loss, asthma, chronic obstructive pulmonary disease, coughing, pulmonary hypertension, optic neurodegenerative diseases (for example, glaucoma, progressive diabetic retinopathy, age-related maculopathy, and retinitis pigmentosa), diabetes mellitus, preterm labor · threatened premature delivery, functional dyspepsia, irritable bowel syndrome, and the like.

The compound I is preferably suitable for preventing and/or treating dysuria.

The compound I is more preferably suitable for preventing and/or treating overactive bladder.

The overactive bladder is a symptom syndrome which includes urinary urgency as an essential symptom, and is generally accompanied by frequent urination and nocturia, and is sometimes accompanied by urge urinary incontinence.

The compound I may be administered as a concomitant drug by being combined with another drug for

1) complementing and/or enhancing the prophylactic and/or therapeutic effect,
2) improving the kinetics and absorption and reducing the dose, and/or
3) reducing adverse effects.

The concomitant drug of the compound I and one or more types of other drugs may be administered in the form of a combination drug in which all the components are combined in a single preparation or may take a form in which the components are formulated into separate preparations and administered. In the case where the components are formulated into separate preparations and administered, simultaneous administration and administration at different times are included. Further, in the case of administration at different times, the compound I may be first administered, and the other drug may be administered thereafter, or the other drug may be first administered, and the compound I may be administered thereafter. The respective administration methods may be the same or different.

A disease on which the concomitant drug exhibits a prophylactic and/or therapeutic effect is not particularly limited and may be a disease on which the prophylactic and/or therapeutic effect of the compound I is complemented and/or enhanced.

Examples of the other drug for complementing and/or enhancing the prophylactic and/or therapeutic effect of the compound I on overactive bladder include (1) muscarinic receptor antagonists (for example, tolterodine, oxybutynin, hyoscyamine, propantheline, propiverine, trospium, solifenacin, darifenacin, imidafenacin, fesoterodine, temiverine, flavoxate, tarafenacin, afacifenacin, THVD-101, THVD-201, etc.), (2) β3-adrenergic receptor agonists (mirabegron, KRP-114V, solabegron, TRK-380, etc.), (3) NK-1 or -2 antagonists (for example, aprepitant, cizolirtine, etc.), (4) recombinant botulinum toxins (senrebotase, etc.), (5) opioid μ receptor agonists (TRK-130, etc.), (6) α4β2-nicotinic acetylcholine receptor antagonists (dexmecamylamine, etc.), (7) C-fiber inhibitors (besipirdine, etc.), (8) TRPV1 antagonists (XEN-D0501, etc.), (9) EP1 antagonists (KEA-0447, etc.), (10) central nervous drugs (REC-1819, etc.), (11) α1-adrenergic receptor antagonists (for example, tamsulosin, silodosin, naftopidil, urapidil, etc.), (12) 5α-reductase inhibitors (dutasteride, finasteride, etc.), (13) phosphodiesterase-5 inhibitors (sildenafil, tadalafil, and vardenafil), (14) vasopressin V2 receptor agonists (desmopressin), and the like.

The dose of the other drug can be appropriately selected based on the clinically used dose. Further, the combining ratio of the compound I to the other drug can be appropriately selected according to the age and body weight of an administration target, an administration method, an administration period, a target disease, symptoms, combination, etc. For example, the other drug may be used in an amount of 0.01 to 100 parts by mass with respect to 1 part by mass of the compound I. As the other drug, arbitrary two or more drugs may be combined at an appropriate ratio and administered. Further, in the other drug, not only drugs which have been found so far, but also drugs which will be found in future are also included.

In order to use the compound I or the concomitant drug of the compound I and the other drug for the above-mentioned purpose, it is generally formulated into an appropriate pharmaceutical composition together with a pharmaceutically acceptable carrier and then administered systemically or topically in the form of an oral or parenteral preparation.

The compound I is administered to a mammal (preferably, a human, more preferably, a patient) in a pharmaceutically effective amount.

The dose of the compound I is dependent on the age, body weight, symptoms, a desired therapeutic effect,

an administration route, a treatment period, etc., and therefore inevitably varies. In general, the compound I is orally administered at a dose ranging from 0.1 mg to 1000 mg once to several times a day per patient, or parenterally administered at a dose ranging from 0.01 mg to 100 mg once to several times a day per patient, or continuously administered intravenously for a period ranging from 1 hour to 24 hours a day.

Of course, as described above, the dose varies depending on various conditions, and therefore, an amount smaller than the above-mentioned dose is sufficient in some cases, or an amount exceeding the range is needed in some cases.

When the compound I or the concomitant drug of the compound I and the other drug is administered, it is used as an oral solid preparation or an oral liquid preparation for oral administration, a sustained-release preparation for oral administration, a controlled-release preparation, or an injection, a topical preparation, an inhalant, or a suppository for parenteral administration, or the like.

The crystal form of the present invention is used as a drug substance of the above-mentioned pharmaceutical products.

## EXAMPLES

Hereinafter, the present invention will be described in detail by way of Examples, however, the present invention is not limited thereto.

The solvents in parentheses shown in the chromatographic separation part and TLC indicate the used elution solvents or development solvents, and the ratio represents a volume ratio.

In the present invention, as a silica gel column chromatograph, Chromatorex (registered trademark), manufactured by Fuji Silysia Chemical Ltd., Yamazen Hi-Flash column (trade name), or the like was used, and as a purification device, for example, a medium-pressure preparative chromatograph W-prep 2XY (trade name), manufactured by the Yamazen Corporation was used.

Unless otherwise specified, the NMR data is $^{1}$H-NMR data.

The solvents used in the measurement are indicated in parentheses shown in the NMR part.

The compound names used in this specification were named using a computer program ACD/Name (registered trademark) of Advanced Chemistry Development, Inc. that generally performs naming according to IUPAC rules or named according to IUPAC nomenclature.

### Example 1

2-(4-bromophenyl)-1,1,1-trifluoro-2-propanol

Under an argon atmosphere, cerium chloride (51 g) dried by heating under reduced pressure was suspended in tetrahydrofuran (316 mL), and the suspension was stirred at room temperature for 1 hour and then cooled to -70°C. Methyl lithium (a 3.0 M diethyl ether solution, 185 mL) was added dropwise thereto, followed by stirring at - 70°C for 30 minutes, and then, a tetrahydrofuran (30 mL) solution of 1-(4-bromophenyl)-2,2,2-trifluoroethanone (40 g) (CAS Registry Number: 16184-89-7) was added thereto, followed by stirring at room temperature for 1.5 hours. The reaction solution was poured into a mixed solution of saturated aqueous ammonium chloride solution (500 mL) and ice water (500 mL), and thereafter, 1 N hydrochloric acid was added thereto until the color of the mixture became light yellow, and then, extraction was performed with ethyl acetate. The organic layer was sequentially washed with water and a saturated aqueous sodium chloride solution, and dried over anhydrous magnesium sulfate, and then concentrated under reduced pressure, whereby the title compound having the following physical property values was obtained (51 g).

TLC: Rf 0.59 (hexane:ethyl acetate = 4:1);

$^{1}$H-NMR ($CDCl_3$): $\delta$ 1.77, 2.42, 7.44-7.47, 7.52-7.55.

### Example 2

(2S)-2-(4-bromophenyl)-1,1,1-trifluoro-2-propanyl[(1S)-1-(1-naphthyl)ethyl]carbamate

To a dichloromethane (237 mL) solution of the compound (43 g) produced in Example 1, dimethylaminopyridine (23.2 g) and 4-nitrophenyl chloroformate (35.1 g) were added under ice-cooling, followed by stirring at room temperature for 1 hour. The reaction solution was ice-cooled again, and (1S)-1-(1-naphthyl)ethylamine (33.2 mL) (CAS Registry Number: 10420-89-0) was added thereto, followed by stirring at room temperature for 1 hour. To the reaction mixture, tert-butyl methyl ether (500 mL) was added, and the deposit was separated by filtration and washed with hexane/ethyl acetate (1/1). The filtrate and the washing solution were combined, and the combined solution was concentrated to about half the amount under reduced pressure, and thereafter sequentially washed with a 1 N aqueous sodium hydroxide

solution (150 mL × 4 times), 1 N hydrochloric acid (200 mL), water (150 mL), and a saturated aqueous sodium chloride solution, and dried over anhydrous magnesium sulfate, and then concentrated under reduced pressure. The resulting residue was purified by silica gel column chromatography (hexane:ethyl acetate = 90:10 → 80:20), whereby a diastereomeric mixture of the title compound was obtained (70 g). The diastereomeric mixture was separated and purified by silica gel column chromatography (hexane:tert-butyl methyl ether = 80:20), whereby the title compound having the following physical property values was obtained (33 g).
TLC: Rf 0.58 (hexane:diisopropyl ether = 2:1);
$^1$H-NMR ($CDCl_3$): δ 1.69, 2.17, 5.25, 5.49-5.58, 7.22-7.25, 7.43-7.58, 7.80-7.95.

Example 3

(2S)-2-(4-bromophenyl)-1,1,1-trifluoro-2-propanol

To a 1,4-dioxane (540 mL) solution of the compound (50 g) produced in Example 2, a solution of lithium hydroxide monohydrate (45 g) in water (270 mL) was added, followed by stirring at 55°C for 1 hour. After the reaction mixture was cooled to 10°C, 2 N hydrochloric acid (540 mL) was added thereto to adjust the pH to 3, and extraction was performed with ethyl acetate. The organic layer was sequentially washed with an aqueous sodium hydrogen carbonate solution, water, an aqueous ammonium chloride solution, and a saturated aqueous sodium chloride solution, and dried over anhydrous magnesium sulfate, and then concentrated under reduced pressure. The resulting residue was purified by silica gel column chromatography (hexane:ethyl acetate = 90:10 → 80:20), whereby the title compound having the following physical property values was obtained (27 g).
TLC: Rf 0.59 (hexane:ethyl acetate = 4:1);
$^1$H-NMR ($CDCl_3$): δ 1.78, 2.42, 7.43-7.56.

Example 4

2-methyl-2-propanyl{4-[(2S)-1,1,1-trifluoro-2-hydroxy-2-propanyl]phenyl}carbamate

A mixture of the compound (30g) produced in Example 3, tert-butyl carbamate (17 g), palladium(II) acetate (2.5 g), 4,5-bis(diphenylphosphino)-9,9-dimethylxanthene (9.7 g), cesium carbonate (55 g), and 1,4-dioxane (225 mL) was stirred under an argon atmosphere at 100°C for 1.5 hours. After the reaction mixture was cooled to room temperature, water (250 mL) and ethyl acetate (250 mL) were added thereto and the resulting mixture was filtered through Celite (trade name). To the filtrate, water (250 mL) was added to separate the mixture into two layers, and the aqueous layer was extracted with ethyl acetate. The organic layers were combined, and the combined solution was sequentially washed with an aqueous ammonium chloride solution and a saturated aqueous sodium chloride solution, and dried over anhydrous magnesium sulfate, and then concentrated under reduced pressure. The resulting residue was purified by silica gel column chromatography (hexane:ethyl acetate = 90:10 → 20:80), whereby the title compound having the following physical property values was obtained (31 g).
TLC: Rf 0.31 (hexane:ethyl acetate = 4:1);
$^1$H-NMR ($CDCl_3$): δ 1.52, 1.77, 2.56, 6.57, 7.37-7.40, 7.48-7.51.

Example 5

(2S)-2-(4-aminophenyl)-1,1,1-trifluoro-2-propanol

To a dichloromethane (200 mL) solution of the compound (31 g) produced in Example 4, trifluoroacetic acid (102 mL) was added, followed by stirring at room temperature for 2 hours. To the reaction mixture, toluene was added, and the mixture was concentrated under reduced pressure. To the resulting residue, a saturated aqueous sodium hydrogen carbonate solution (300 mL) was added, and extraction was performed with ethyl acetate. The organic layer was sequentially washed with an aqueous ammonium chloride solution and a saturated aqueous sodium chloride solution, and dried over anhydrous magnesium sulfate, and then concentrated under reduced pressure. The resulting residue was dissolved in tert-butyl methyl ether-hexane (1:1, 60 mL) at 60°C, and the solution was cooled to 5°C, and the deposited solid was filtered out. The filtrate was concentrated, whereby the title compound having the following physical property values was obtained (15 g).
TLC: Rf 0.37 (hexane:ethyl acetate = 3:2);
$^1$H-NMR ($CDCl_3$): δ 1.74, 2.38, 3.77, 6.67-6.70, 7.33-7.36.

Example 6

(2S)-2-(4-aminophenyl)-1,1,1-trifluoro-2-propanol hydrochloride

After a tert-butyl methyl ether (127 mL) solution of the compound (13 g) produced in Example 5 was cooled in an ice-water bath, a 4 N hydrogen chloride/1,4-dioxane solution (19 mL) was added thereto, followed by stirring. The resulting deposit was collected by filtration and washed with tert-butyl methyl ether. To the obtained solid, acetonitrile (200 mL) was added and dissolved therein by stirring at 80°C, and then, the resulting solution was cooled to room temperature and stirred overnight. The deposited crystal was collected by filtration, and sequentially washed with acetonitrile and then dried, whereby the title compound having the following physical property values was obtained (12.7 g).
TLC: Rf 0.34 (hexane:ethyl acetate = 3:2);
$^1$H-NMR (DMSO-$d_6$): $\delta$ 1.64, 3.57, 6.61, 7.19-7.22, 7.58-7.61.

Example 7

4-{(2S)-1,1,1-trifluoro-2-[(trimethylsilyl)oxy]-2-propanyl}aniline

The compound (12.7 g) produced in Example 6 was dissolved in methanol (10 mL) and ethyl acetate (70 mL), and a saturated aqueous sodium hydrogen carbonate solution (150 mL) was added thereto in divided portions while stirring, and the reaction mixture was extracted with ethyl acetate. The organic layer was washed with a saturated aqueous sodium chloride solution and dried over anhydrous magnesium sulfate, and then concentrated under reduced pressure. To the resulting residue, a tetrahydrofuran (210 mL) solution was added, followed by cooling in an ice-water bath, and thereafter, imidazole (20 g) and chlorotrimethylsilane (33.4 mL) were added thereto, followed by stirring at room temperature for 15 hours. The reaction mixture was poured into water (400 mL), and extraction was performed with ethyl acetate. The organic layer was sequentially washed with water and a saturated aqueous sodium chloride solution and dried over anhydrous magnesium sulfate, and then concentrated under reduced pressure, whereby the title compound having the following physical property values was obtained (14.4 g).
TLC: Rf 0.52 (hexane:ethyl acetate = 2:1);
$^1$H-NMR ($CDCl_3$): $\delta$ 0.12, 1.77, 3.69, 6.64-6.67, 7.29-7.32.

Example 8

2,6-dichloro-4-{(2S)-1,1,1-trifluoro-2-[(trimethylsilyl)oxy]-2-propanyl}aniline

To a N,N-dimethylformamide (144 mL) solution of the compound (14.4 g) produced in Example 7, N-chlorosuccinimide (13.8 g) was added, followed by stirring at room temperature for 15 hours, and then, further stirring at 40°C for 5 hours. The reaction mixture was poured into water (500 mL), and extraction was performed with hexane-ethyl acetate (1:2, 150 mL $\times$ 3 times). The organic layer was washed with a saturated aqueous sodium chloride solution and dried over anhydrous magnesium sulfate, and then concentrated under reduced pressure. The resulting residue was purified by silica gel column chromatography (hexane:ethyl acetate = 90:10 $\rightarrow$ 50:50), whereby the title compound having the following physical property values was obtained (17.4 g).
TLC: Rf 0.56 (hexane:ethyl acetate = 4:1);
$^1$H-NMR ($CDCl_3$): $\delta$ 0.15, 1.75, 4.51, 7.33.

Example 9

M crystal of 1-[(5-chloro-2-pyridinyl)methyl]-3-{2,6-dichloro-4-[(2S)-1,1,1-trifiuoro-2-hydroxy-2-propanyl] phenyl}urea

[Chem. 3]

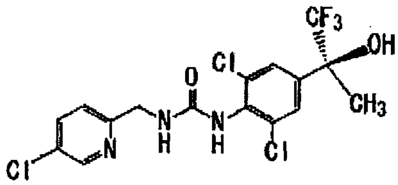

To a tetrahydrofuran (3.6 mL) solution of the compound (250 mg) produced in Example 8, N,N-diisopropylethylamine (137 μL) and triphosgene (235 mg) were added. The reaction mixture was stirred at room temperature for 30 minutes, and then concentrated under reduced pressure. The resulting residue was dissolved in tetrahydrofuran (3.6 mL), and (5-chloropyridin-2-yl)methanamine hydrochloride (186 mg) (CAS Registry Number: 871826-13-0) and triethylamine (241 μL) were added thereto, followed by stirring at room temperature for 1 hour. To the reaction mixture, water was added, and extraction was performed with ethyl acetate. The organic layer was sequentially washed with water and a saturated aqueous sodium chloride solution and dried over anhydrous sodium sulfate, and then concentrated under reduced pressure. To the resulting residue, methanol (3.6 mL) and trifluoroacetic acid (1 mL) were added, followed by stirring at room temperature for 5 hours. The reaction mixture was concentrated under reduced pressure, and thereafter, a saturated aqueous sodium hydrogen carbonate solution was added thereto, and extraction was performed with ethyl acetate. The organic layer was sequentially washed with water and a saturated aqueous sodium chloride solution and dried over anhydrous sodium sulfate, and then concentrated under reduced pressure. The resulting residue was purified by silica gel column chromatography (hexane:ethyl acetate = 60:40 → 10:90), whereby the title compound having the following physical property values was obtained (243 mg). In Examples of the present invention, production is performed by the same production method as in Example 25(5) of Patent Application 2.
TLC: Rf 0.22 (hexane:ethyl acetate = 1:1);
$^1$H-NMR (DMSO-$d_6$): δ 1.69, 4.36, 6.93, 7.06, 7.38, 7.64, 7.94, 8.37, 8.55.
The powder X-ray diffraction spectrum chart and the DSC chart of the crystal measured under the following conditions are shown in FIG. 1 and FIG. 2, respectively.

(1) Powder X-Ray Diffraction Spectrum

Device: SmartLab, manufactured by Rigaku Corporation
Target: Cu
Voltage: 45 kV
Current: 200 mA
Scanning rate: 5 °/min

The results of diffraction angles (2θ) (°) and relative intensities (%) obtained by powder X-ray diffraction spectrometry using a Cu-Kα ray are shown in Table 1.

[Table 1]

Table 1

| Diffraction angle (2θ) (°) | Relative intensity (%) |
|---|---|
| 7.0 | 79 |
| 7.6 | 25 |
| 9.1 | 50 |

(continued)

| Diffraction angle (2θ) (°) | Relative intensity (%) |
|---|---|
| 9.2 | 100 |
| 10.1 | 26 |
| 10.5 | 23 |
| 11.2 | 42 |
| 12.0 | 27 |
| 12.4 | 62 |
| 12.9 | 30 |
| 13.2 | 61 |
| 13.6 | 17 |
| 13.9 | 63 |
| 14.4 | 21 |
| 14.8 | 15 |
| 15.6 | 19 |
| 15.8 | 34 |
| 16.5 | 20 |
| 17.4 | 23 |
| 17.6 | 33 |
| 18.1 | 37 |
| 18.6 | 29 |
| 19.2 | 27 |
| 19.9 | 18 |
| 20.3 | 63 |
| 21.1 | 59 |
| 22.0 | 31 |
| 22.2 | 37 |
| 22.7 | 33 |
| 23.1 | 26 |
| 23.7 | 21 |
| 24.1 | 30 |
| 24.5 | 19 |
| 24.9 | 28 |

(2) Differential Scanning Calorimetry (DSC)

Device: differential scanning calorimeter DSC822e, manufactured by Mettler Toledo International, Inc.
Sample amount: 1.18 mg
Sample cell: Aluminum standard 40 μL
Nitrogen gas flow rate: 40 mL/min
Temperature raising rate: 10 °C/min (25 to 200°C)

In the DSC chart, two endothermic peaks and one exothermic peak were observed. The first endothermic peak is attributed to the melting of the M crystal.

First endothermic peak: onset temperature: 89.8°C, peak temperature: 100.0°C
Second endothermic peak: onset temperature: 157.5°C, peak temperature: 163.8°C
Exothermic peak: onset temperature: 114.2°C, peak temperature: 121.5°C

Example 10

A crystal of 1-[(5-chloro-2-pyridinyl)methyl]-3-12,6-dichloro-4-[(2S)-1,1,1-trifiuoro-2-hydroxy-2-propanyl]phenyl}urea

To the compound (10 mg) produced in Example 9, ethanol (50 μL) was added, and the compound was dissolved therein at 40 to 60°C. A solid obtained by stirring this solution overnight at room temperature was collected by filtration and dried, whereby a crystalline white solid (A crystal) having the following physical property values was obtained.

The powder X-ray diffraction spectrum chart and the DSC chart of the crystal measured under the following conditions are shown in FIG. 3 and FIG. 4, respectively.

(1) Powder X-Ray Diffraction Spectrum

Device: SmartLab, manufactured by Rigaku Corporation
Target: Cu
Voltage: 45 kV
Current: 200 mA
Scanning rate: 5 °/min

The results of diffraction angles (2θ) (°) and relative intensities (%) obtained by powder X-ray diffraction spectrometry using a Cu-Kα ray are shown in Table 2.

[Table 2]

Table 2

| Diffraction angle (2θ) (°) | Relative intensity (%) |
|---|---|
| 11.3 | 25 |
| 11.6 | 42 |
| 11.9 | 45 |
| 12.8 | 38 |
| 13.9 | 49 |
| 15.3 | 60 |
| 16.4 | 14 |
| 16.9 | 33 |
| 17.4 | 30 |
| 18.1 | 18 |
| 19.3 | 77 |
| 19.6 | 20 |
| 20.5 | 81 |
| 21.1 | 100 |

(continued)

| Diffraction angle (2θ) (°) | Relative intensity (%) |
|---|---|
| 22.2 | 12 |
| 22.8 | 19 |
| 23.5 | 98 |
| 23.8 | 57 |
| 24.3 | 18 |
| 24.7 | 19 |

(2) Differential Scanning Calorimetry (DSC)

Device: differential scanning calorimeter DSC822e, manufactured by Mettler Toledo International, Inc.

Sample amount: 1.8 mg

Sample cell: Aluminum standard 40 μL

Nitrogen gas flow rate: 40 mL/min

Temperature raising rate: 10 °C/min (25 to 180°C)

In the DSC chart, an endothermic peak (onset temperature: 166.0°C, peak temperature: 167.3°C) attributed to the melting of the A crystal was observed.

Example 11

W crystal of 1-[(5-chloro-2-pyridinyl)methyl]-3-{2,6-dichloro-4-[(2S)-1,1,1-trifluoro-2-hydroxy-2-propanyl]phenyl}urea

To the compound (10 mg) produced in Example 9, methanol (30 to 40 μL) was added, and the compound was dissolved therein at 40 to 60°C. A solid obtained by stirring this solution overnight at room temperature was collected by filtration and dried, whereby a crystalline white solid (W crystal) having the following physical property values was obtained.

The powder X-ray diffraction spectrum chart and the DSC chart of the crystal measured under the following conditions are shown in FIG. 5 and FIG. 6, respectively.

(1) Powder X-Ray Diffraction Spectrum

Device: SmartLab, manufactured by Rigaku Corporation
Target: Cu
Voltage: 45 kV
Current: 200 mA
Scanning rate: 20 °/min

The results of diffraction angles (2θ) (°) and relative intensities (%) obtained by powder X-ray diffraction spectrometry using a Cu-Kα ray are shown in Table 3.

[Table 3]

Table 3

| Diffraction angle (2θ) (°) | Relative intensity (%) |
|---|---|
| 10.6 | 85 |
| 15.2 | 100 |
| 19.6 | 87 |
| 20.7 | 73 |
| 22.4 | 70 |
| 24.1 | 66 |

(2) Differential Scanning Calorimetry (DSC)

Device: differential scanning calorimeter DSC822e, manufactured by Mettler Toledo International, Inc.
Sample amount: 0.71 mg
Sample cell: Aluminum standard 40 μL
Nitrogen gas flow rate: 40 mL/min
Temperature raising rate: 10 °C/min (25 to 180°C)

In the DSC chart, two endothermic peaks and one exothermic peak were observed. The first endothermic peak is attributed to the melting of the W crystal.

First endothermic peak: onset temperature: 88.6°C, peak temperature: 94.3°C
Second endothermic peak: onset temperature: 165.1°C, peak temperature: 167.0°C
Exothermic peak: onset temperature: 113.0°C, peak temperature: 119.0°C

Example 12

Crystal of hydrate (H crystal) of 1-[(5-chloro-2-pyridinyl)methyl]-3-{2,6-dichloro-4-[(2S)-1,1,1-trifluoro-2-hydroxy-2-propanyl]phenyl}urea

To the compound (20 mg) produced in Example 10, water (400 μL) was added, followed by stirring at 25 to 40°C for 15 days to one month, and the resulting solid was collected by filtration, whereby a crystalline white solid (H crystal) having the following physical property values was obtained.
The powder X-ray diffraction spectrum chart and the DSC chart of the crystal measured under the following conditions are shown in FIG. 7 and FIG. 8, respectively.

(1) Powder X-Ray Diffraction Spectrum

Device: SmartLab, manufactured by Rigaku Corporation
Target: Cu
Voltage: 45 kV
Current: 200 mA
Scanning rate: 5 °/min

The results of diffraction angles (2θ) (°) and relative intensities (%) obtained by powder X-ray diffraction spectrometry using a Cu-Kα ray are shown in Table 4.

[Table 4]

Table 4

| Diffraction angle (2θ) (°) | Relative intensity (%) |
|---|---|
| 6.5 | 27 |
| 8.6 | 23 |
| 11.4 | 73 |
| 11.7 | 42 |
| 13.0 | 100 |
| 14.5 | 52 |
| 15.1 | 28 |
| 16.3 | 30 |
| 18.3 | 36 |
| 19.0 | 55 |
| 19.4 | 83 |
| 20.1 | 62 |
| 20.5 | 75 |
| 21.7 | 61 |
| 21.9 | 25 |
| 22.3 | 44 |
| 23.0 | 29 |
| 23.5 | 91 |
| 23.8 | 38 |
| 24.2 | 28 |
| 24.5 | 24 |

(2) Differential Scanning Calorimetry (DSC)

Device: differential scanning calorimeter DSC822e, manufactured by Mettler Toledo International, Inc.
Sample amount: 0.68 mg
Sample cell: Aluminum standard 40 μL
Nitrogen gas flow rate: 40 mL/min
Temperature raising rate: 10 °C/min (25 to 180°C)

In the DSC chart, two endothermic peaks were observed. The first endothermic peak is attributed to the dehydration of the hydrate (H crystal).

First endothermic peak: onset temperature: 31.7°C, peak temperature: 48.2°C
Second endothermic peak: onset temperature: 165.3°C, peak temperature: 166.8°C

Subsequently, the results of the water vapor adsorption/desorption isotherm and the X-ray diffraction-differential scanning calorimetry simultaneous measurement (XRD-DSC simultaneous measurement) of the H crystal are shown in FIG. 9 and FIG. 10, respectively.

(3) Water Vapor Adsorption/Desorption Isotherm Measurement

Device: DVS Intrinsic, manufactured by SMS Co., Ltd.
Sample amount: 5.5 mg
Temperature: 25°C
Change in relative humidity: 0% to 95% (5% STEP)
STEP transition criterion: percent change in weight: 0.002%/min

A change in mass when the humidity was changed in a stepwise manner (relative humidity: from 0% RH to 95% RH) at 25°C was recorded over time, and an equilibrium mass at each humidity was determined. Subsequently, based on the mass when dried (0% RH), the percent change in mass at each humidity and the hydration number were determined.
The results are shown in FIG. 9. When the relative humidity was increased in a stepwise manner, a change in mass was observed at around 30%, and the equilibrium was reached when the relative humidity was 50% RH, and the percent change in mass at a relative humidity of 50% RH or more was constantly 4%. In the case where the relative humidity was decreased in a stepwise manner subsequently thereto, the percent change in mass was constantly 4% up to around 15%. The theoretical water content of a monohydrate is 4%, and therefore, the results of this test indicate a possibility that the H crystal is a monohydrate.

(4) Powder X-Ray Diffraction-Differential Scanning Calorimetry Simultaneous Measurement (PXRD-DSC simultaneous measurement)

Device: SmartLab, manufactured by Rigaku Corporation
Target: Cu
Voltage: 45 kV
Current: 200 mA
Scanning rate: 20 °/min
Temperature raising rate: 2 °C/min (room temperature to 150°C)

At around 40 to 50°C, an endothermic peak accompanying dehydration, and a change in pattern in the X-ray diffraction spectrum chart accompanying a change in crystal structure were observed.

Comparative Example 1

1-[4-(1,1,1-trifluoro-2-hydroxy-2-propanyl)phenyl]-3-{[5-(trifluoromethyl)-2-pyridinyl]methyl}urea

[Chem. 4]

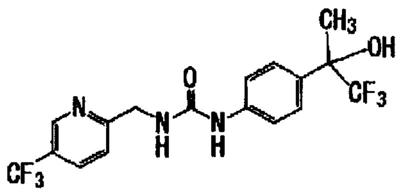

The title compound having the following physical property values was obtained by performing the same procedure as in Comparative Example 1 of Patent Application 2.

TLC: Rf 0.25 (hexane:ethyl acetate = 1:1);
$^1$H-NMR (DMSO-$d_6$): δ 1.63, 4.49, 6.42, 6.86, 7.41, 7.57, 8.18, 8.90.

Comparative Example 2

N-(2-bromo-4,6-dichlorophenyl)-2-(4-fluorophenyl)acetamide (Example 1g of Patent Document 1)

[Chem. 5]

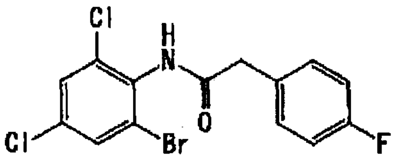

The title compound (1.28 g) having the following physical property values was obtained by performing the same procedure as in Comparative Example 2 of Patent Application 2.
TLC: Rf 0.43 (hexane:ethyl acetate = 1:1);
$^1$H-NMR ($CD_3OD$): δ 3.72, 7.02-7.08, 7.38-7.43, 7.58, 7.69.
The effects of the compound I can be demonstrated by the following experiments, but is not limited thereto.

(1) Biological Example 1: Opening Action with respect to KCNQ2/3 Channel by Depolarization Stimulation

Human KCNQ2/3 expression cells (CHO-DHFR- cells) were seeded in each well of a 384-well plate (collagen-coated, black, clear bottom) at 0.5 × $10^4$ cells/50 μL per well and cultured in a MEM ALPHA medium (containing 10 vol% inactivated (56°C, 30 min) fetal bovine serum, 100 IU/mL penicillin, 100 μg/mL streptomycin, and 2 mM L-glutamine) at 37°C in 5% CO2 for 18 to 24 hours. After the medium in the plate was removed, incubation (room temperature, 60 minutes, shading) was performed in a loading buffer (prepared by the method described in the manual of FluxOR Thallium Detection Kit (Invitrogen, F10016, F10017)). A KCNQ2/3 channel opening action (thallium influx into cells) by depolarization stimulation (5 mM potassium and 0.5 mM thallium) was measured by FLIPR TETRA (Molecular Devices). The compound I was treated 5 minutes before the depolarization stimulation, and the reaction induced by the depolarization stimulation was measured over time for 180 seconds. The channel opening action of the compound I was evaluated based on the change amount of the fluorescence intensity from the time before the depolarization stimulation to the time after the elapse of 180 seconds, and the concentration (ECrtg50) satisfying 50% of the fluorescence intensity change of a maximum reaction (in the treatment at 10 μM) of retigabine under the conditions of this experiment was calculated.
As a result, the opening action (ECrtg50 value) of the compound I with respect to the KCNQ2/3 channel was 0.6 μM. On the other hand, the ECrtg50 value of Comparative Example 1 was >10 μM, and the ECrtg50 value of Comparative Example 2 was 0.2 μM.
Further, in the above-mentioned method, by using human KCNQ4 or human KCNQ5 expression cells in place of the human KCNQ2/3 expression cells and appropriately changing the above-mentioned conditions based on the ordinary knowledge of those skilled in the art, the opening action with respect to the human KCNQ4 channel or the human KCNQ5 channel can be measured.

(2) Biological Example 2: Relaxing Action on Bladder

Extracted from Rat

Female Jcl:Wistar rats (CLEA Japan, Inc., body weight at use: 170 to 200 g) were anesthetized by intraperitoneal administration of about 40 mg/kg of pentobarbital (Somnopentyl, Schering Plough Animal Health Corporation), and killed by bloodletting. The bladder was extracted by abdominal incision and immediately thereafter immersed in ice-cooled Krebs buffer (Krebs Ringer bicarbonate buffer (Sigma-Aldlich Co. LLC) supplemented with sodium hydrogen carbonate (final concentration: 15 mM) and calcium chloride (final concentration: 2.5 mM)) saturated with a mixed gas (95% O2, 5% $CO_2$).

The bladder body extracted from each rat was cut into an oblong strip shape (about 10 × 3 mm), whereby a specimen was prepared on ice. Immediately thereafter, the specimen was suspended in a Magnus tube filled with Krebs buffer (37°C) bubbled with the mixed gas while applying a tension load of 500 mg thereto. Incidentally, the specimen was prepared within 24 hours after extracting the tissue.

The change in tension of the specimen was recorded in data collection system (NR-1000, KEYENCE CORPORATION) through a Magnus system equipped with an isometric transducer (UFER UM-203, Iwashiya Kishimoto Medical Instruments) and an amplifier (UFER AP-5, Iwashiya Kishimoto Medical Instruments) and displayed on a computer via recorder analysis software WAVE THERMO 1000 (KEYENCE CORPORATION). After the elapse of one hour or more from the suspension of the specimen, 2.5 M KCl was added thereto to a final concentration of 100 mM, and the specimen in which a contraction reaction could be confirmed was used.

Carbachol (a contraction inducer) contraction was induced at a concentration of 1 μM. The specimens were arbitrarily assigned in groups so that a difference in the degree of contraction did not occur among the groups and the specimens collected from the same individual did not belong to the same group. After the contraction reaction was stabilized, physiological saline or the compound I was added thereto to a final concentration of 1 nM, 10 nM, 100 nM, 1 μM, and 10 μM in a cumulative manner from a low concentration.

The tension (mg) in the extracted bladder was used as an evaluation item. The tension was read using analysis software WAVE THERMO 1000. A percent change in tension after adding the compound I when the tension after adding the contraction inducer was taken as 0% was defined as a percent change in tension (%) and used as an evaluation index. The percent change in tension (%) is calculated according to the following formula.

$$\text{percent change in tension (\%)} = \{\text{tension (mg) after adding compound I and the like} - \text{tension (mg) before adding contraction inducer}\}/\{\text{tension (mg) after adding contraction inducer (mg)} - \text{tension before adding contraction inducer}\} \times 100 - 100$$

The value at which the percent change in tension (%) was -20% was calculated as IC20 and used as an index of the relaxing action on the extracted bladder.

As a result, the $IC_{20}$ value in the rat Magnus test of the compound I was 0.5 μM. The compound I has a relaxing action on the bladder extracted from the rat. Therefore, the compound I is useful as a therapeutic agent for overactive bladder.

(3) Solubility Test

A calibration curve solution was prepared at 0.1, 0.4, and 2 μM by diluting a test substance (a 10 mM DMSO solution) with acetonitrile and adding acetonitrile containing an internal standard substance (candesartan).

A sample solution was prepared as follows. 5 μL of the compound I (a 10 mM DMSO solution) was added to 495 μL of the Japanese Pharmacopoeia dissolution test second solution (pH: 6.8), followed by stirring at room temperature for 5 hours. Then, the solution was transferred to a solubility filter plate and subjected to suction filtration. The filtrate (20 μL) was diluted with acetonitrile, followed by adding acetonitrile containing an internal standard substance (candesartan).

Each of the calibration curve and sample solutions (5 μL) was injected into LC-MS/MS (Discovery Max, manufactured by Thermo Scientific, Inc.) and subjected to quantification (quantification range: 5 to 100 μM). The solubility in the case where the value equal to or less than the quantification range was obtained was defined as <5 μM, and the solubility in the case where the value equal to or more than the quantification range was obtained was defined as 100 μM.

As a result, the compound I exhibited an excellent solubility (98 μM). On the other hand, the solubility of Comparative Example 2 (Example 1g of Patent Document 1) was equal to or less than the detection limit (<5 μM), and the solubility of Comparative Example 2 was low.

(4) Evaluation of Stability in Human Liver Microsomes

A test compound (a 10 mmol/L DMSO solution, 5 μL) was diluted with a 50% aqueous acetonitrile solution (195 μL), whereby a 0.25 mmol/L solution was prepared.

To a reaction vessel preheated to 37°C, 0.5 mg/mL of human liver microsomes (XenoTech, LLC) and 245 μL of a 0.1 M phosphate buffer solution (pH 7.4) containing NADPH co-factor (BD Biosciences) were added, followed by preincubation for 5 minutes, and thereafter, the above-prepared test compound solution (5 μL) was added thereto to start a reaction (final concentration: 5 μmol/L). Immediately after the start, a 20 μL portion was collected and added to 180 μL of acetonitrile containing an internal standard substance (warfarin) to stop the reaction. A 20 μL portion of this

solution was stirred with 180 μL of a 50% aqueous acetonitrile solution on a plate equipped with a deproteinization filter, followed by suction filtration, and the filtrate was used as a standard sample.

After the above-prepared reaction solution was incubated at 37°C for 15 minutes, a 20 μL portion of the solution was added to 180 μL of cooled acetonitrile (containing warfarin serving as an internal standard substance) to stop the reaction. A 20 μL portion of this solution was stirred with 180 μL of a 50% aqueous acetonitrile solution on a plate equipped with a deproteinization filter, followed by suction filtration, and the filtrate was used as a reaction sample.

A residual ratio (%) was calculated as follows. 1 μL of the sample solution was injected into LC-MS/MS (Discovery Max, manufactured by Thermo Scientific, Inc.), and a value obtained by dividing the peak area ratio of the reaction sample (peak area of test compound/peak area of internal standard substance) by the peak area ratio of the standard sample was multiplied by 100.

As a result, it was found that the compound I has high stability (87%) against the human liver microsomes, and has excellent metabolic stability.

(5) Evaluation of Action on hERG IKr Current

By using HEK293 cells overexpressing a human ether-a-go-go-related gene (hERG), the maximum tail current of the hERG IKr current induced by redepolarization pulse subsequent to depolarization pulse was measured by a patch-clamp method. The percent change (suppression rate) after 10 minutes from application of a test substance with respect to the maximum tail current before the application of the test substance was calculated (see, Biophysical Journal, vol. 74, pp. 230-241 (1998)). As a result, the 50% hERG channel inhibitory activity of the compound I is > 10 μM, and it was found that the compound I is a compound which has a low possibility of inducing QT extension due to a drug, and therefore has excellent safety.

(6) Chemical Stability Test

The stability of the crystal form of the present invention was examined under various storage conditions. After storage, the residual ratio (%) of each of the samples stored under the respective conditions with respect to the area percentage of the sample stored at -20°C was calculated by HPLC. Further, by using a powder X-ray diffraction spectrum, peaks were compared with those of the sample. The appearance was observed by visual inspection and compared with that of the sample.

<Storage Conditions and Sampling Time>

5°C: 3 months
60°C: 1 month
25°C-60% RH (opened): 3 months
40°C-75% RH (opened): 3 months
2500 Lux: 20D (shading, transparent)

<A Crystal>

Under any conditions, the residual ratio was 99.9% to 100.1%. Further, in the examination using a powder X-ray diffraction spectrum, an increase in the number of peaks was not observed, and also a change in appearance was not observed by visual inspection. Therefore, it was found that the A crystal is a crystal form having excellent chemical stability.

[Formulation Examples]

Representative formulation examples to be used in the present invention will be shown below.

Formulation Example 1

The following respective components are mixed by a conventional method, followed by tableting, whereby 10,000 tablets containing 10 mg of the active ingredient per tablet are obtained:

- A crystal of 1-[(5-chloro-2-pyridinyl)methyl]-3-{2,6-dichloro-4-[(2S)-1,1,1-trifluoro-2-hydroxy-2-propanyl]phenyl}urea

(100 g);

- carboxymethylcellulose calcium (disintegrating agent) (20 g);
- magnesium stearate (lubricant) (10 g); and
- microcrystalline cellulose (870 g).

Formulation Example 2

The following respective components were mixed by a conventional method, followed by filtration through a dust removal filter, and a 5 mL portion of the filtrate was filled into ampoules, and the ampoules were subjected to heat sterilization in an autoclave, whereby 10,000 ampoules containing 20 mg of the active ingredient per ampoule are obtained:

- A crystal of 1-[(5-chloro-2-pyridinyl)methyl]-3-{2,6-dichloro-4-[(2S)-1,1,1-trifluoro-2-hydroxy-2-propanyl]phenyl}urea (200 g);
- mannitol (2 kg); and
- distilled water (50 L).

INDUSTRIAL APPLICABILITY

The compound I has sufficiently low toxicity, and can be used safely as a pharmaceutical product and is useful as a therapeutic agent for a KCNQ2-5 channel-related disease. Further, the crystal form of the present invention is useful as a drug substance of a pharmaceutical product.

## Claims

**1.** A crystal of 1-[(5-chloro-2-pyridinyl)methyl]-3-{2,6-dichloro-4-[(2S)-1,1,1-trifluoro-2-hydroxy-2-propanyl]phenyl} urea, having at least peaks of about 15.3, 16.9, 17.4, 19.3, and 19.6 °2θ in a powder X-ray diffraction spectrum.

**2.** The crystal of 1-[(5-chloro-2-pyridinyl)methyl]-3-(2,6-dichloro-4-[(2S)-1,1,1-trifluoro-2-hydroxy-2-propanyl]phenyl} urea according to claim 1, having no peaks of about 7.0 and 9.2 °2θ in a powder X-ray diffraction spectrum.

**3.** The crystal of 1-[(5-chloro-2-pyridinyl)methyl]-3-{2,6-dichloro-4-[(2S)-1,1,1-trifluoro-2-hydroxy-2-propanyl]phenyl} urea according to claim 1 or 2, having peaks of about 11.3, 11.6, 11.9, 12.8, 13.9, 15.3, 16.4, 16.9, 17.4, 18.1, 19. 3, 19.6, 20.5, 21.1, 22.2, 22.8, 23.5, 23.8, 24.3, and 24.7 °2θ in a powder X-ray diffraction spectrum.

**4.** The crystal of 1-[(5-chloro-2-pyridinyl)methyl]-3-{2,6-dichloro-4-[(2S)-1,1,1-trifluoro-2-hydroxy-2-propanyl]phenyl} urea according to any one of claims 1 to 3, **characterized by** a powder X-ray diffraction spectrum chart shown in FIG. 3.

**5.** A crystal of 1-[(5-chloro-2-pyridinyl)methyl]-3-{2,6-dichloro-4-[(2S)-1,1,1-trifluoro-2-hydroxy-2-propanyl]phenyl} urea, having an endothermic peak with an onset temperature of about 166°C or a peak temperature of about 167°C in differential scanning calorimetry.

**6.** The crystal of 1-[(5-chloro-2-pyridinyl)methyl]-3-{2,6-dichloro-4-[(2S)-1,1,1-trifluoro-2-hydroxy-2-propanyl]phenyl} urea according to claim 5, **characterized by** a differential scanning calorimetry chart shown in FIG. 4.

**7.** A crystal of a hydrate of 1-[(5-chloro-2-pyridinyl)methyl]-3-{2,6-dichloro-4-[(2S)-1,1,1-trifluoro-2-hydroxy-2-propanyl]phenyl}urea.

**8.** The crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to claim 7, having at least peaks of about 13.0, 14.5, 18.3, 19.0, and 21.7 °2θ in a powder X-ray diffraction spectrum.

**9.** The crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to claim 8, having no peaks of about 7.0 and 9.2 °2θ in a powder X-ray diffraction spectrum.

**10.** The crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to claim 8 or 9, having peaks of about 6.5, 8.6, 11.4, 11.7, 13.0, 14.5, 15.1, 16.3, 18.3, 19.0, 19.4, 20.1, 20.5, 21.7, 21.9, 22.3, 23.0, 23.5, 23.8, 24.2, and 24.5 °2θ in a powder X-ray diffraction spectrum.

**11.** The crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to any one of claims 8 to 10, **characterized by** a powder X-ray diffraction spectrum chart shown in FIG. 7.

**12.** The crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to claim 7, having an endothermic peak with an onset temperature of about 32°C or a peak temperature of about 48°C in differential scanning calorimetry.

**13.** The crystal of a hydrate of 1-[(5-chloro-2-pyridinyl)methyl]-3-{2,6-dichloro-4-[(2S)-1,1,1-trifluoro-2-hydroxy-2-propanyl]phenyl}urea according to claim 12, **characterized by** a differential scanning calorimetry chart shown in FIG. 8.

**14.** A pharmaceutical composition comprising the crystal according to any one of claims 1 to 13 and a pharmaceutically acceptable carrier.

**15.** The pharmaceutical composition according to claim 14, which is a prophylactic and/or therapeutic agent for a KCNQ2-5 channel-related disease.

**16.** The pharmaceutical composition according to claim 15, wherein the KCNQ2-5 channel-related disease is dysuria.

**17.** The pharmaceutical composition according to claim 16, wherein the dysuria is overactive bladder.

**18.** A prophylactic and/or therapeutic agent for a KCNQ2-5 channel-related disease, comprising the crystal according to any one of claims 1 to 13.

**19.** A method for preventing and/or treating a KCNQ2-5 channel-related disease, **characterized by** administering an effective amount of the crystal according to any one of claims 1 to 13 to a mammal.

**20.** The crystal according to any one of claims 1 to 13 for preventing and/or treating a KCNQ2-5 channel-related disease.

**21.** Use of the crystal according to any one of claims 1 to 13 for manufacturing a prophylactic and/or therapeutic agent for a KCNQ2-5 channel-related disease.

Fig. 1

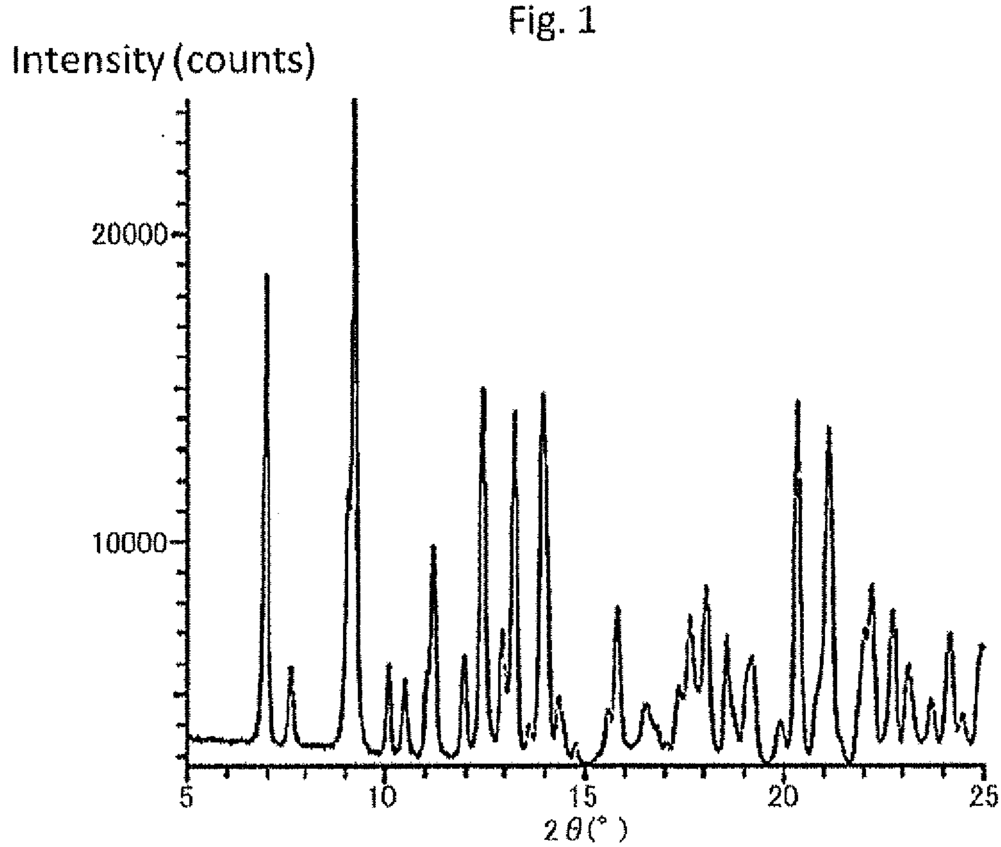
Intensity (counts)
20000
10000
5
10
15
20
25
2θ(°)

Fig. 2

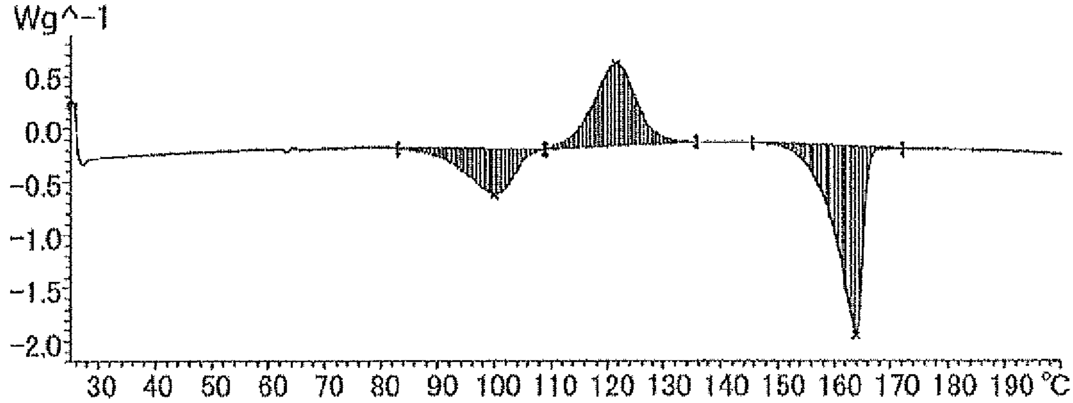
Wg^-1
0.5
0.0
-0.5
-1.0
-1.5
-2.0
30 40 50 60 70 80 90 100 110 120 130 140 150 160 170 180 190 °C

Fig. 3

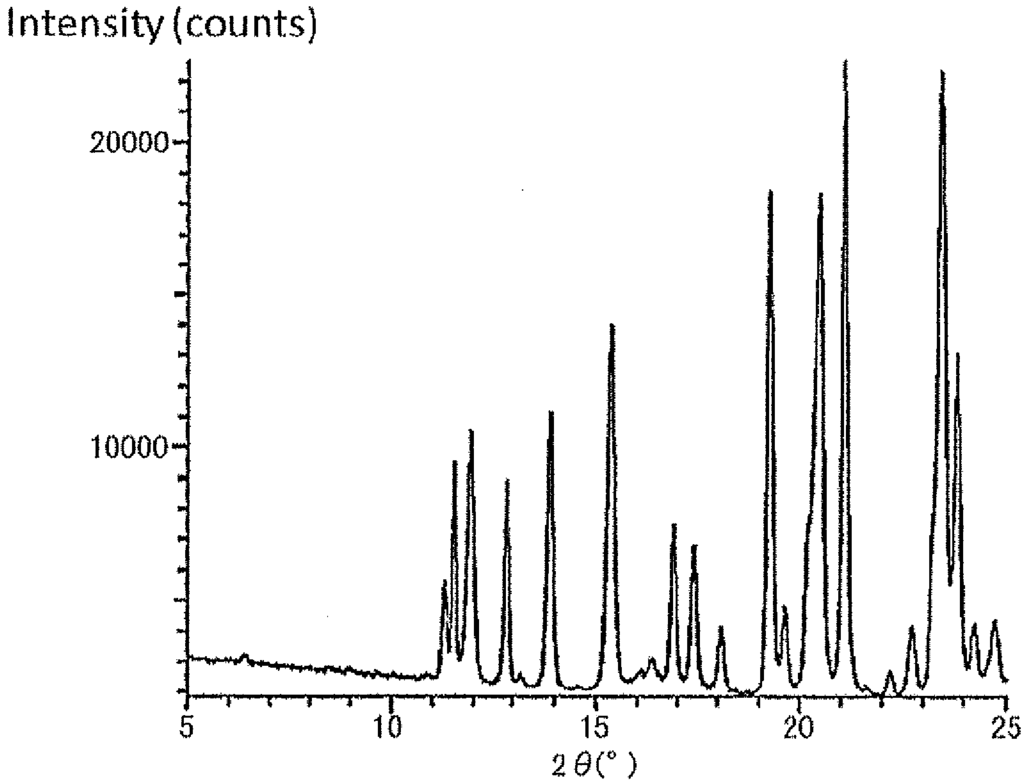
Intensity (counts)
20000
10000
5
10
15
20
25
2θ(°)

Fig. 4

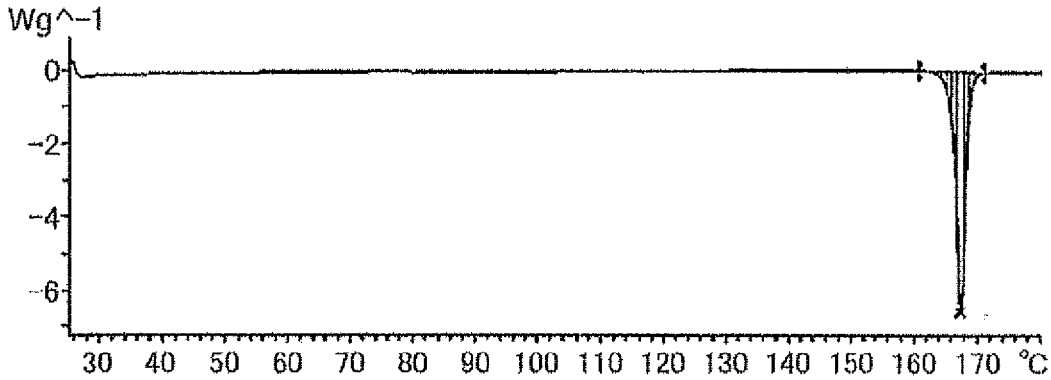
Wg^-1
0
-2
-4
-6
30 40 50 60 70 80 90 100 110 120 130 140 150 160 170 °C

Fig. 5

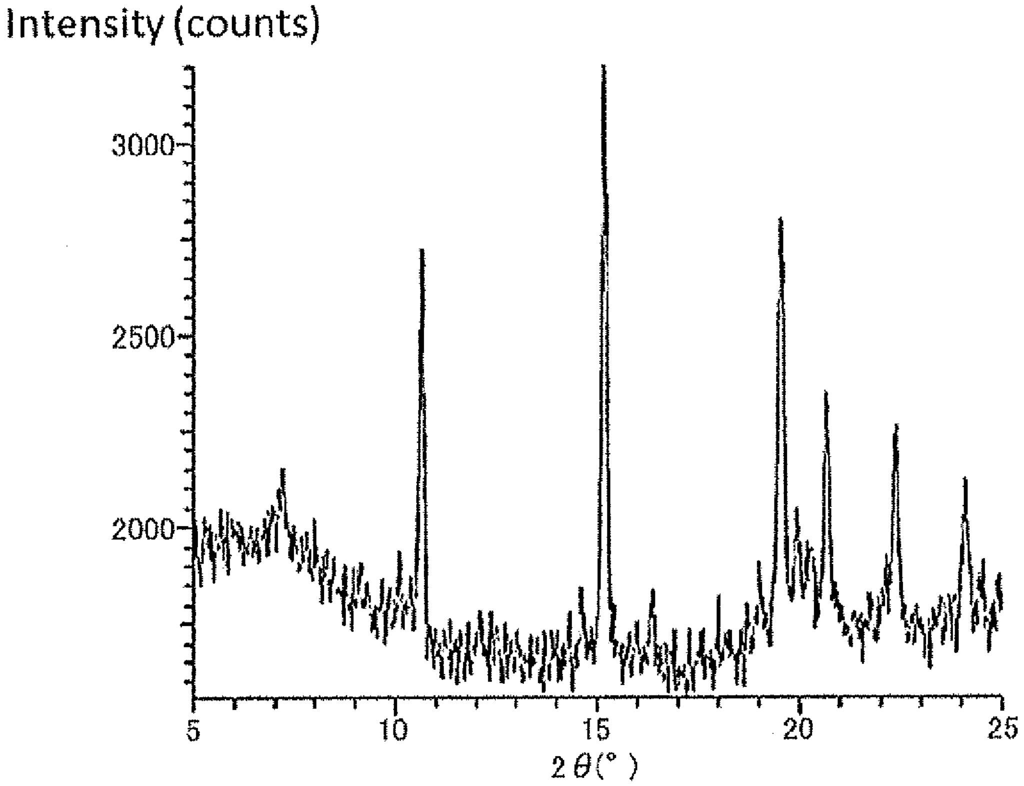
Intensity (counts)
3000
2500
2000
5
10
15
20
25
2θ(°)

Fig. 6

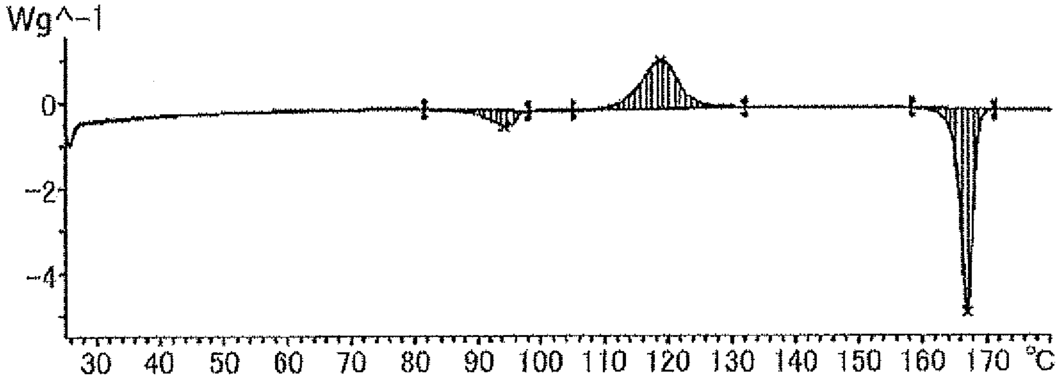
Wg^-1
0
-2
-4
30 40 50 60 70 80 90 100 110 120 130 140 150 160 170 °C

Fig. 7

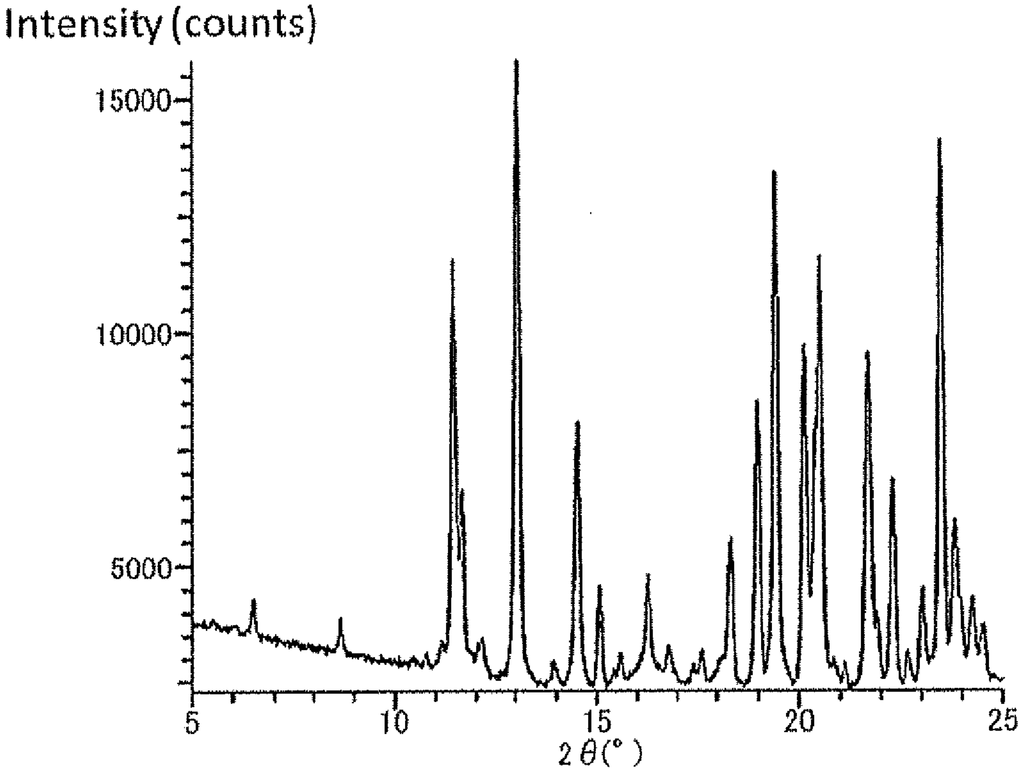
Intensity (counts)
15000
10000
5000
5
10
15
20
25
2θ(°)

Fig. 8

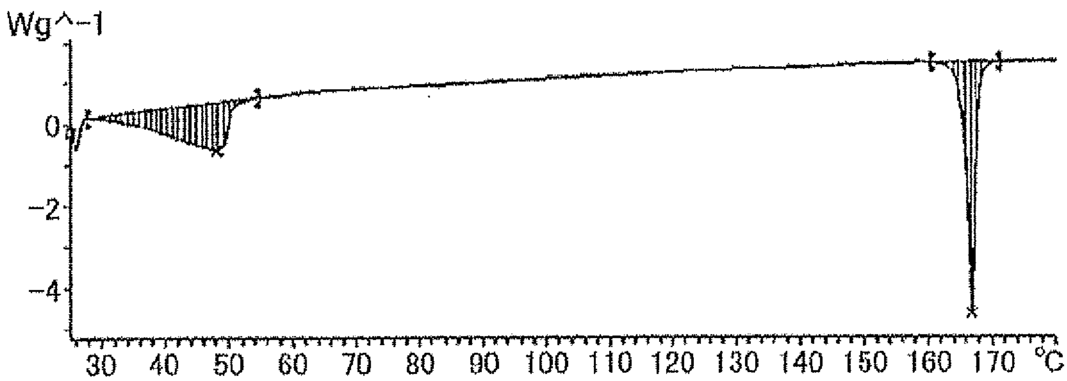
Wg^-1
0
-2
-4
30
40
50
60
70
80
90
100
110
120
130
140
150
160
170
°C

Fig. 9

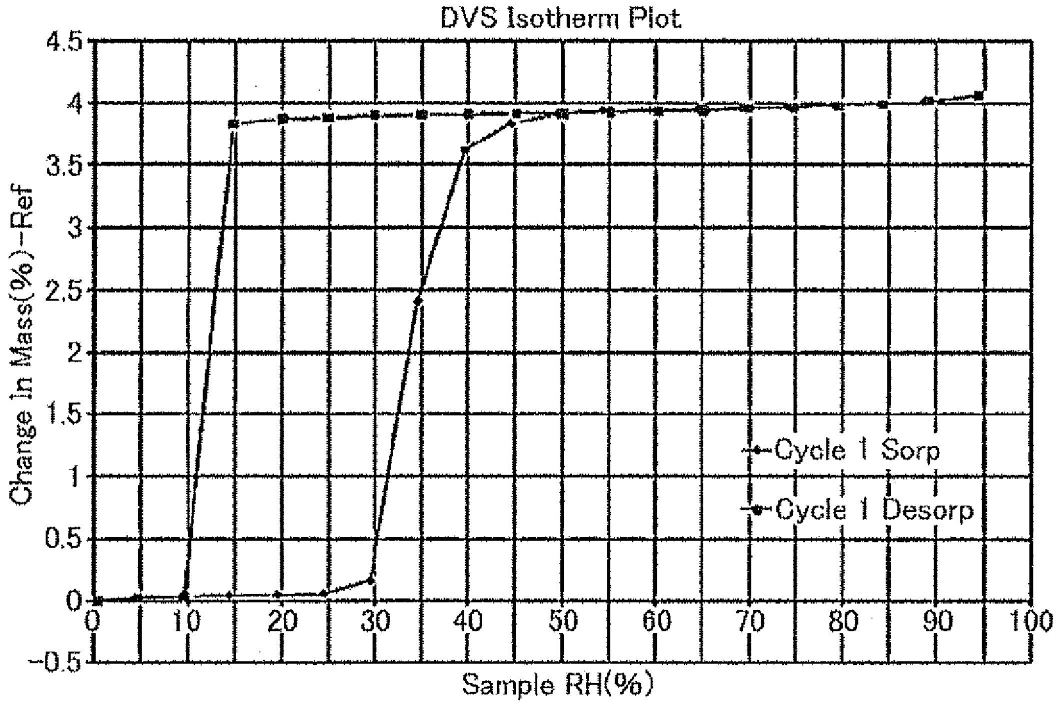
DVS Isotherm Plot
Change in Mass(%)-Ref
Sample RH(%)
Cycle 1 Sorp
Cycle 1 Desorp
4.5
4
3.5
3
2.5
2
1.5
1
0.5
0
-0.5
0
10
20
30
40
50
60
70
80
90
100

Fig. 10

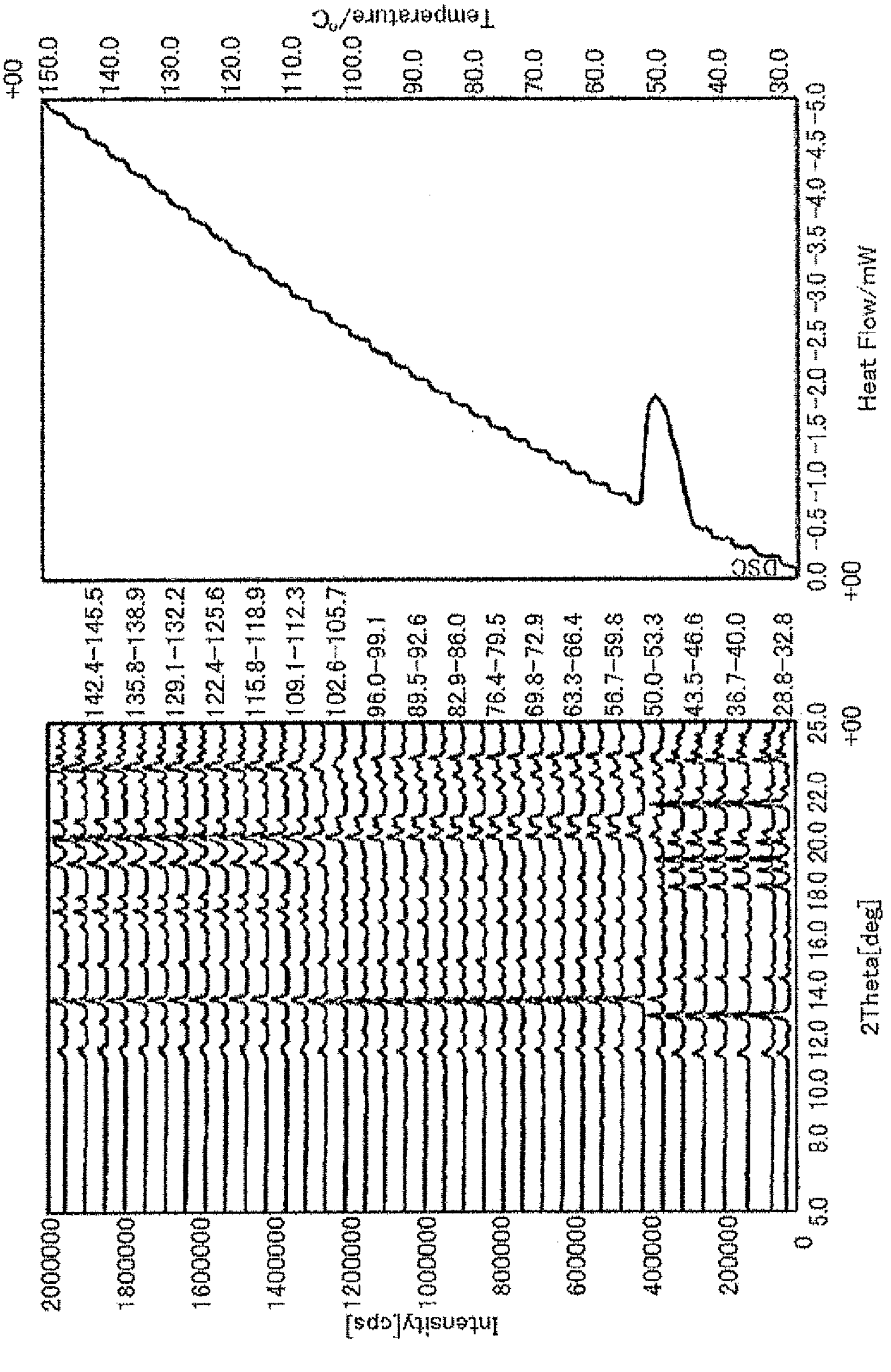
Temperature/°C
Heat Flow/mW
DSC
2Theta[deg]
Intensity[cps]
142.4–145.5
135.8–138.9
129.1–132.2
122.4–125.6
115.8–118.9
109.1–112.3
102.6–105.7
96.0–99.1
89.5–92.6
82.9–86.0
76.4–79.5
69.8–72.9
63.3–66.4
56.7–59.8
50.0–53.3
43.5–46.6
36.7–40.0
28.8–32.8

**INTERNATIONAL SEARCH REPORT**

International application No. PCT/JP2017/016109

A. CLASSIFICATION OF SUBJECT MATTER
*C07D213/61*(2006.01)i, *A61K31/44*(2006.01)i, *A61P13/10*(2006.01)i, *A61P43/00*(2006.01)i

According to International Patent Classification (IPC) or to both national classification and IPC

B. FIELDS SEARCHED

Minimum documentation searched (classification system followed by classification symbols)
C07D213/61, A61K31/44, A61P13/10, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| P,X | WO 2016/063990 A1 (Ono Pharmaceutical Co., Ltd.), 28 April 2016 (28.04.2016), particularly, example 25(5) & CA 2965467 A & AU 2015336458 A & TW 201625533 A | 1-21 |
| A | Yusaku SHIOJI, Manufacture Technology of Solid Tablet, trade edition, Tokyo: CMC Publishing Co., Ltd., 27 January 2003 (27.01.2003), pages 9, 12 to 13 | 1-21 |
| A | BYRN,S. et al., Pharmaceutical Solids: A Strategic Approach to Regulatory Considerations, Pharmaceutical Research, 1995, Vol.12, No.7, pp.945-954 | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 July 2017 (07.07.17) | 18 July 2017 (18.07.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/016109

C (Continuation). DOCUMENTS CONSIDERED TO BE RELEVANT

| Category* | Citation of document, with indication, where appropriate, of the relevant passages | Relevant to claim No. |
|---|---|---|
| A | Akira OGATA, Kagaku Jikken Sosaho First Volume, 27th edition, Nankodo Co., Ltd., 20 November 1963 (20.11.1963), pages 366 to 399 | 1-21 |
| A | Jikken Kagaku Koza (Zoku) 2 Bunri to Seisei, Maruzen Co., Ltd., 25 January 1967 (25.01.1967), pages 159 to 162, 184 to 193 | 1-21 |
| A | The Chemical Society of Japan, Jikken Kagaku Guidebook, 3rd edition, Maruzen Co., Ltd., 30 April 1992 (30.04.1992), pages 130 to 131 | 1-21 |
| A | JP 2016-508960 A (Gruenenthal GmbH), 24 March 2016 (24.03.2016), entire text & US 2014/0148468 A1 & WO 2014/082737 A1 & EP 2951156 A1 & CA 2892745 A & AU 2013351550 A | 1-21 |
| A | JP 2015-514728 A (Gruenenthal GmbH), 21 May 2015 (21.05.2015), entire text & WO 2013/156154 A1 & EP 2888233 A1 & CA 2870573 A & AU 2013248637 A & AR 90739 A & EA 201401142 A & MX 2014012494 A & CN 104428287 A & HK 1210173 A | 1-21 |
| A | JP 2010-535244 A (Valeant Pharmaceuticals International), 18 November 2010 (18.11.2010), entire text & US 2009/0170885 A1 & WO 2009/018466 A1 & EP 2183250 A1 & EP 2311836 A1 & DE 602008004555 D & AU 2008282118 A & CA 2695196 A & KR 10-2010-0068373 A & CN 101778848 A & MX 2010001189 A & AT 495175 T & ES 2359601 T & RU 2010107284 A & TW 200922571 A | 1-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016063990 A
- WO 2006029623 A


### Non-patent literature cited in the description


- *Current Topics in Medicinal Chemistry,* 2006, vol. 6, 999-1023
- *The Journal of Urology,* 2004, vol. 172, 2054-2058
- *European Journal of Pharmacology,* 2010, vol. 638, 121-127
- *Biophysical Journal,* 1998, vol. 74, 230-241